# EUROPEAN PATENT SPECIFICATION

(11) **EP 3 062 853 B1**
(45) Date of publication and mention of the grant of the patent: **23.10.2019**
(21) Application number: 14858221.6
(22) Date of filing: 30.10.2014
(51) Int. Cl.: A61M 11/00, A61M 15/00, A61M 16/20, B05B 7/00, A61M 11/06, A61M 16/10, A61M 16/08

(54) **IMPROVEMENTS TO SMALL-VOLUME NEBULIZERS**
VERBESSERUNGEN AN KLEINVOLUMIGEN VERNEBLERN
PERFECTIONNEMENTS APPORTÉS À DES NÉBULISEURS DE PETIT VOLUME

(30) Priority: 30.10.2013 US 201314067045
(43) Date of publication of application: 07.09.2016
(73) Proprietor: Caddo Medical Technologies LLC, Dallas, TX 75214 (US)
(72) Inventor: Faram, Joseph Dee, Dallas, TX 75214 (US)
(74) Representative: Carstens, Dirk Wilhelm
(86) International application number: PCT/US2014/063030
(87) International publication number: WO 2015/066256

(56) References cited:
- WO-A1-2011/080761
- US-A- 4 253 468
- US-A- 4 951 661
- US-A- 5 012 804
- US-A- 5 027 809
- US-A- 5 337 962
- US-A- 5 357 945
- US-A- 5 364 615
- US-A- 5 813 401
- US-A1- 2002 162 554
- US-A1- 2006 021 613
- US-A1- 2008 066 754
- US-A1- 2008 283 050
- US-A1- 2009 272 820
- US-A1- 2011 209 700
- US-B1- 6 412 481
- US-B1- 6 422 234
- US-B1- 6 510 846

## Description

### FIELD OF THE INVENTION

The invention relates to small-volume nebulizers and components associated therewith.

### BACKGROUND OF THE INVENTION

It is estimated that more than thirty million people each year are treated for respiratory diseases such as asthma and cystic fibrosis by aerosolizing medication in disposable, small-volume nebulizers, following which the medicine is then inhaled by a patient as a part of the patient's therapy. Bronchodilators, such as albuterol sulfate or ipratropium bromide, are typically used in order to improve airflow among patients with pulmonary maladies. Additional medicines, used in different forms of therapy or to treat different maladies, are also possible. As used herein, the terms "medicine" and "medication" shall refer to any one or a combination of substances used primarily in patient treatment and specifically excluding substances such as saline solution or water used primarily for the humidification of gases inhaled by a patient.

Pharmaceutical companies originally packaged these medicines in containers that held multiple doses. In order to initiate a patient treatment, the medicine needed to be transferred from the container to the treatment equipment such as a nebulizer. As the containers were repeatedly opened and closed, the medicine was exposed to bacterial contamination. In order to stem bacterial growth, chemicals such as benzalkonium chloride, or BAC, were added. However, it was eventually found that BAC itself may lead to airway constriction. See, Meyer, Harriet, "Antibacterial Agent In Some Asthma Medications Linked To Airway Constriction, UF Scientists Find." UF News, Jan 11, 2001. Thus, the use of BAC may have negated or at least reduced any positive effect the bronchodilators may have had.

In order to reduce bacterial contamination without adding potentially harmful antibacterial chemicals, pharmaceutical manufacturers began to package respiratory drugs in single-dose or "unit-dose" containers, thus removing the need to repeatedly open a container of medicine to dispense a dose. These unit-dose respiratory drugs are typically packaged in soft plastic containers often formed from low density polyethylene, or LDPE, in order to help control costs and to make the containers easy to open.

Typically, the medication is opened by twisting the top of the unit-dose container until the plastic gives way at a thin portion of plastic at the neck. The medication is then transferred into a disposable nebulizer by aiming the unit-dose container opening at the nebulizer housing opening, squeezing the soft plastic of the container until the contents have emptied, and then disposing of the empty unit-dose container.

However, unit-dose packaging was found to have inherent drawbacks. First, packaging costs increased over the previous bulk packaging due to the fact that each dose necessitated its own container. Second, the mere fact that the medicine must be transferred from a packaging container to a nebulizer or other treatment device is believed to carry an inherent risk of contamination. Further, it was found that LDPE is permeable to chemicals that have moderate to high vapor pressure, such as adhesives, varnishes, inks, and solvents, all of which are typically used in labeling and packaging materials. After it was determined that a number of different inhalation drugs packaged in LDPE unit-dose containers were contaminated with these chemicals, the industry moved away from printed paper-and-ink labels to embossed labeling with raised lettering. See, Grissinger, Matthew, "Errors in the Making: Nearly Unreadable Labeling of Plastic Ampules for Nebulizing Agents." Medication Errors; P&T Journal May 2005; Vol. 30, No. 5, pp. 255-58.

Unfortunately, medication errors due to the poor legibility of embossed lettering on LDPE unit-dose containers have caused great concern in the medical community. See, Grissinger, Id. Drug names, concentrations, lot numbers, and expiration dates are embossed into the containers in the form of transparent, raised letters rendering them virtually impossible to read. This leads all too frequently to administering the wrong drug. Mistakes occur when unit-dose respiratory drugs are stored in refrigerated "respiratory bins" where a number of different drugs are typically placed. The risk of using the wrong medication is also increased when clinicians keep various unit-dose medications in their laboratory coat pockets, which is often the case.

The problem of potential medication errors associated with embossed labeling on unit-dose containers continues. Transferring medication from unit-dose containers takes time, adds to difficulty of use, introduces the potential for contamination during transfer, and runs the risk of under-dosing due to spillage. In addition, there still remains the added packaging cost associated with packaging each dose separately, not to mention environmental concerns associated with the disposal of millions of plastic unit-dose containers. Finally, even though LDPE plastic containers are more malleable than other plastics, these containers are still difficult to open, especially for elderly and arthritic patients.

Thus, there remains a need for packaging system for liquid medicines, which may be clearly labeled without risk of label-chemical contamination, which reduces the risk of contamination during transfer of medication from container to nebulizer, which reduces or eliminates the cost associated with each dose needing its own individual container, which saves the time associated with transferring medication from container to nebulizer, which reduces the need for disposal of millions of plastic unit-dose containers, which reduces the risk of under-dosing due to spillage, and which may still be more easily opened or used by elderly and arthritic patients.

Medical nebulizers are divided into two general categories: 1) large-volume, and 2) small-volume. Large-volume nebulizers are used, most often in hospital settings, to humidify gas, usually oxygen, to a patient. Large-volume nebulizers are utilized to add moisture to otherwise very dry gas by aerosolizing water, usually sterilized water with some mixture of saline in order to mimic the human body's salt content. Large-volume nebulizers often come pre-filled with various mixtures of sterile water and saline. Large-volume nebulizers have a reservoir volume greater than 100 ml. See Bruce K. Rubin & James B. Fink, Aerosol Therapy for Children, in 7 No. 2 RESPIRATORY CARE CLINICS OF NORTH AMERICA: AEROSOL THERAPY, 175, 187 (James B. Fink & Rajiv Dhand eds. 2001).

Small-volume nebulizers, also referred to as "hand-held nebulizers," are used for delivering medication to the lungs. Small-volume nebulizers are powered by high-pressure air or oxygen and are classified as pneumatic jet nebulizers. These devices are used for aerosolized medication therapy in both home and hospital settings. Although small-volume nebulizers are utilized in the delivery of a number of medications from analgesics to antibiotics, they are most often used to administer bronchodilators. Because they are for drug administration rather than humidification, small-volume nebulizers have medication reservoirs of 10 to 15 ml or less.

Small-volume nebulizers have come under scrutiny in recent years because of bacterial contamination. Traditionally, it has been common practice to clean and reuse disposable, single-patient-use, small-volume nebulizers. However, unless the nebulizer is completely sterilized it has been found that these "cleaned" nebulizers run the risk of growing such pathogens as *Pseudomonas aeruginosa, Staphylococcus aureus,* and *Haemophilus influenzae,* as well as other dangerous organisms. It is believed that contamination of the nebulizer occurs not only in spite of the cleaning, but may indeed be due to the cleaning itself. It is thought that poor cleaning techniques, inadequate drying, and the use of potable water sources may contribute to the contamination. Because of the risk of contamination and the fact that small-volume nebulizers are relatively inexpensive, especially when compared to the cost of nosocomial infections, many hospitals have come to the conclusion that it is safer and more prudent to dispose of the small-volume nebulizer soon after use. For example, it is currently a practice in many hospitals to utilize the same disposable nebulizer for twenty-four hours without cleaning, and then to dispose of it. *See*, O'Malley, Catherine A, et al. "A Day in the Life of a Nebulizer: Surveillance for Bacterial Growth in Nebulizer Equipment of Children With Cystic Fibrosis in the Hospital Setting." Respiratory Care 2007, Vol. 52, No. 3, pp. 258-62.

Respiratory patients are often treated with physiotherapies, physical therapies without medication, for the purposes of opening the airways, assisting in increased bilevel flow to enhance secretion movement, and to strengthen diaphragm muscles. These therapies generally fit into one of two categories depending upon the source of flows and pressures utilized in the therapy. One such category of devices utilizes flow and pressure generated by an external apparatus, such as intermittent positive pressure breathing devices (IPPB) such as the Mark 7®, MetaNeb®, or The Vest®. Each of these devices mechanically generates the flows and pressures necessary for the therapy. This category of device is more costly and intended for patients with a higher acuity level, many of whom do not have the ability to supply their own flows and pressures sufficiently to be effective.

The other category utilizes the flow and pressure generated by the patient. Examples of device in this category are positive expiratory pressure, Aerobika™, and Flutter®. Therapies that depend on flows and pressures created by the patient use either constant or intermittent resistance to the patient's flow. Positive expiratory pressure (PEP) is a constant resistance to patient exhalation, keeping backpressure on airways to keep them open. Some PEP devices also oscillate and are known as "oscillating positive expiratory pressure" (OPEP). Flutter® is another form of OPEP. The devices in this category are usually less costly and intended for those patients with less acuity and still strong enough to supply their own flow for the therapy.

The demands of the healthcare industry require devices and methods that deliver therapies appropriate to the patient's acuity, which cost less, require less time, and maximize efficacy. To this end, there are problems with the scarcity of choices among existing devices and methods for delivering these therapies.

Some small-volume nebulizers generate great variability in particle sizes, or heterodispersal, during the conversion of liquid medication into aerosol. Since various particle sizes tend to deposit in different locations within the lungs, it is desirable to reduce a wide variability of particle sizes to a tighter, more consistent size-range range, making the aerosol more monodispersed.

In addition, small-volume nebulizers waste medication during the exhalation phase of the breathing cycle. The medication is lost during a treatment in relation to the person's inspiratory to expiratory (I:E) ratio which typically ranges from 1:2 to 1:3. That is, if a patient has an I:E ratio of 1:2, for every 1 second the patient inhales 2 seconds are required to exhale. Accordingly, a small-volume nebulizer that creates continuous aerosol only delivers 1 second, or 1/3, of that aerosol to the patient and the remaining 2 seconds, or 2/3, is exhaled into the atmosphere. This waste of medication serves to further escalate the overall cost of healthcare to society.

Some research appears to indicate that healthcare workers, especially nurses and respiratory therapists, have a higher incidence of breathing maladies, such as asthma, than do other professionals. There is concern that these clinicians who work in environments where the delivery of aerosolized medications is commonplace may be put at risk by being forced to breathe the otherwise wasted medication mentioned above that remains suspended in ambient air.

Attempts have been made to create small-volume nebulizers that produce aerosol only during the inspiratory phase of the breath by utilizing complex valve systems that divert a high-pressure source gas away from the aerosolizing chamber. Other attempts to reduce the loss of medication include adding a series of one-way valves and additional reservoirs to retain the aerosol being produced during the patient's expiratory phase so that it can be delivered in a large bolus and the beginning of the next inspiratory phase of breath. Examples of these attempts are to be seen in a breathing circuit apparatus with a 50 ml reservoir (US 5 584 285 A) and a reservoir bag apparatus (US 6 390 090 B1). However, the complexity of design increases the number parts to be manufactured as well as increasing the labor of assembly, thus increasing the cost of the device from existing small-volume nebulizers. In addition, the additional reservoirs and/or reservoir volume present the problem of additional surface area to which the aerosol can adhere, thus still wasting medication. In addition, the increased complexity and components may increase the likelihood of malfunctions in the operation of the device.

Another problem with delivering medication and non-medication therapies is the paucity of single devices that have the ability to deliver multiple therapies inexpensively. If a patient is deemed to require both medicated aerosol therapy and PEP physiotherapy typically two separate devices must be used. The need to use multiple devices to deliver the therapies results in added cost, added training time, added storage space, and may lead to an increased likelihood of contamination.

Attempts to combine physiotherapy devices and nebulizers, such as an oscillating PEP device (US 8 539 951 B1) and a small-volume nebulizer designed to manufacture aerosol only during inspiration (US 6 044 841 A), require significant complexity and resulting high cost. In addition, the increased complexity may increase the likelihood of malfunctions in the operation of the device.

Further attention is drawn to US 2002 / 162554 A1 disclosing a valved aerosol tee adapter assembly with a horizontal portion having an inlet port and an outlet port and a vertical portion including a spring valve that is biased closed until a nebulizer is inserted forcing the valve open. This spring valve is operable only by mechanical force applied to a valve actuator.

Moreover, US 6 412 481 B1 discloses a sealed backpressure attachment device for nebulizer. The device includes a nebulizer attached to a tube having a mouthpiece on one end and a conduit having an inlet one-way valve on the other end. A separate exhalation one-way valve also extends off the tube between the mouthpiece and the inlet one-way valve.

Furthermore, US 5 813 401 A discloses a nebulizer automatic control valve. The device is in a "T" shape and includes a vertically disposed feed tube which connects to the output of a nebulizer and a horizontally disposed breath tube across the top of the feed tube. An internal shuttle cylinder is located within the horizontal breath tube and moves in the breath tube.

Finally, US 4 951 661 A discloses a quick-connect adapter valve for connecting nebulizer and fluid ventilator hose. The adapter is designed to fit into a "T" connector's vertical port. The adapter includes a spring valve that is biased closed until a nebulizer is inserted forcing the valve open. This spring valve is operable only by mechanical force applied to the valve's spokes.

The present invention is directed to solving one or more of the above stated problems that are associated with the cited art.

### SUMMARY OF THE INVENTION

In accordance with the present invention, a valve system apparatus for attachment to a small-volume nebulizer as set forth in claim 1 is provided. Further embodiments of the invention are inter alia disclosed in the dependent claims. The present disclosure relates to small-volume nebulizers, components for use with small-volume nebulizers.

In some embodiments, the present disclosure relates to a small-volume nebulizer pre-filled with medication so that the nebulizer may also serve as a medication container. It is comprised of a small-volume nebulizer containing medication, hermetically sealed, with removable caps at the top and bottom ports.

Some embodiments may include a valve system for a small-volume nebulizer comprised of at least one valve gate that opens and closes in response to a patient's breathing cycle. This valve system is placed at the top of the aerosolizing chamber at either the top or bottom of the chimney, between the patient opening and the aerosolizing chamber of the small-volume nebulizer.

In some embodiments, the present disclosure includes a means for choking the flow of the patient's inspiratory and expiratory effort. The means of choking patient flow also serves to deliver physiotherapy to the airways.

In some embodiments, the valve system and/or the means for choking the flow may be incorporated into the small-volume nebulizer. In some embodiments, the valve system and/or the means for choking the flow may be incorporated into a component that is attachable to the chimney of the small-volume nebulizer.

In some embodiments, the small-volume nebulizers include the nebulizer body or aerosolizing chamber, which includes a medication reservoir, a jet, a siphon and a baffle, a source of high-pressure gas, and an aerosol outlet port or chimney. High-pressure gas passes through the jet, which is a restricted orifice, and then flows through a siphon immersed in the medication, which is held in the medication reservoir. The siphoned medication is propelled at a high speed against a baffle, which is a surface that serves to break up the liquid medication into various sizes and cause larger aerosol particles to fall out of suspension.

In some embodiments, a "T" piece is attached to the aerosol outlet port or chimney. The "T" piece may include an airflow choking mechanism and a patient interface port or mouthpiece. In some embodiments, the aerosol output may include a valve system operational with a patient's breathing cycle.

In some embodiments, the improved small-volume nebulizer increases ease of use and time savings by eliminating a step in the procedure of administering aerosol medication.

In some embodiments, the improved small-volume nebulizer eliminates or reduces the costs associated with both disposable medicine containers and disposable nebulizers.

In some embodiments, the improved small-volume nebulizer eliminates or reduces the likelihood of contaminating medication during transfer of medication from a storage container to a treatment device such as a nebulizer.

In some embodiments, the improved small-volume nebulizer reduces the environmental burden associated with the disposal of unit-dose plastic containers and disposable nebulizers.

In some embodiments, the improved small-volume nebulizer reduces medication identity and volume dosing errors.

In some embodiments, the improved small-volume nebulizer reduces under-dosing due to spillage.

In some embodiments, the improved small-volume nebulizer increases the ease of opening medicine containers.

In some embodiments, the improved small-volume nebulizer reduces storage space required for both respiratory medications and small-volume nebulizers.

In some embodiments, the improved small-volume nebulizer minimizes egress of aerosolized medication from the aerosolizing chamber into the atmosphere during a patient's exhalation in order to reduce the waste of medication.

In some embodiments, the improved small-volume nebulizer reduces the flow of exhaled aerosolized medication into the atmosphere in order to further protect others from inhaling the medication for whom it is not intended.

In some embodiments, the improved small-volume nebulizer allows medication, which may escape during exhalation to be additionally filtered in order to further protect others from inhaling the medication for whom it is not intended.

In some embodiments, the improved small-volume nebulizer provides a non-drug, physiotherapy for the patient by creating inspiratory and expiratory flow resistance during a small-volume nebulizer treatment.

In some embodiments, the improved small-volume nebulizer targets the deposition of medication into particular regions of the lungs by reducing the heterodispersal of aerosol particle sizes.

In some embodiments, the improved small-volume nebulizer suppresses the aerosol being produced in the aerosol chamber from escaping that chamber during exhalation, making the medicated aerosol particles more monodispersed while adding the capability of simultaneous delivery of both physiotherapy and medicated aerosol therapy.

In some embodiments, the improved small-volume nebulizer reduces clinician-training time by reducing the number of devices needed to deliver multiple therapies.

In some embodiments, the improved small-volume nebulizer reduces respiratory storage space by reducing the number of devices needed to deliver multiple therapies.

In some embodiments, the improved small-volume nebulizer reduces costs by combining and simplifying said device, by reducing the number of parts needed to construct said device.

Additional aspects, advantages and features of the present invention are included in the following description of exemplary examples thereof, which description should be taken in conjunction with the accompanying figures, wherein like numerals are used to describe the same feature throughout the figures.

### A BRIEF DESCRIPTION OF THE DRAWINGS

FIG. 1 is a side view of the pre-filled, small-volume nebulizer of present disclosure.
FIG. 2 is a side view of an alternate embodiment of the pre-filled, small-volume nebulizer of present disclosure with a piercable outlet port cap, a one-way valve at the outlet port, a plurality of pre-filled unit-doses of medication, and unit-dose completion demarcation marks.
FIG. 3 is a side view of an alternate embodiment of the pre-filled, small-volume nebulizer of the present disclosure containing a separation compartment for a first component of a multi-component medication housed in the outlet port cap, and a second component of a multi-component medication housed in the nebulizer housing.
FIG. 4 is a side view of a preferred embodiment of the valve system incorporated integrally into a small-volume nebulizer.
FIG. 5 is a side view of the valve system incorporated into a nebulizer "T" piece detachably connected to a small-volume nebulizer.
FIG. 6A is a detailed side view of the valve system with the valve gate center pivot open.
FIG. 6B is a detailed frontal view of the valve system with the valve gate center pivot open.

### DETAILED DESCRIPTION

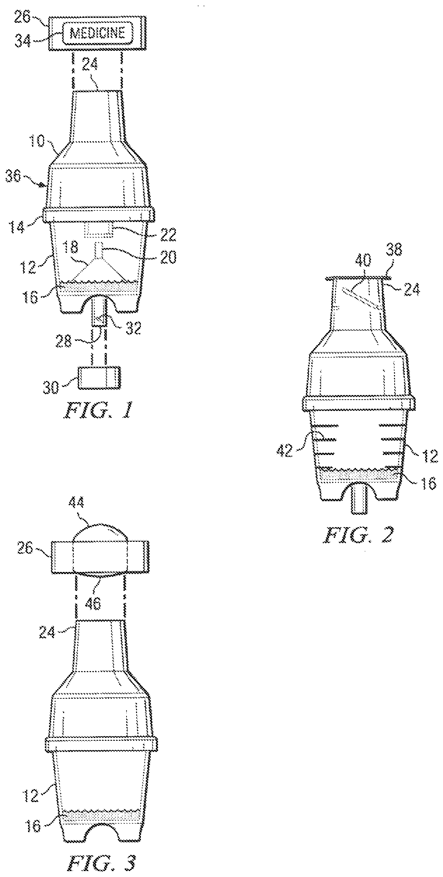

FIG. 1 shows a side view of a preferred embodiment of the pre-filled, small-volume nebulizer of present invention. Each component depicted herein may be fabricated by means of injection molding of a plastic compound, of such material as polypropylene or other plastic compound with appropriate properties for housing medication and fabricating a nebulizer. The pre-filled, small-volume nebulizer may be comprised of a housing top 10, a housing bottom 12, a housing seal 14, pre-filled unit-dose of medication 16, a siphon 18, a jet 20, a baffle 22, an outlet port 24, an outlet port cap 26, an inlet port 28, an inlet port cap 30, an inlet one-way valve 32, an outlet port cap medication label 34.

The general structure and assembly of small volume nebulizers is known in the art. Housing top 10, housing bottom 12, siphon 18, jet 20, baffle 22, outlet port 24, and inlet port 28 are generally cylindrical or conical in shape and are generally co-axial with one another. Baffle 22 and outlet port 24 are typically formed as a part of housing top 10, while inlet port 28 is typically formed as a part of housing bottom 12. Typically, siphon 18 and jet 20 will be formed together in a single piece, with the resulting piece placed inside housing bottom 12. In typical prior art nebulizers, housing top 10 and housing bottom 12 are detachably joined by a threaded connection or by a press fit.

In the nebulizer of the present invention, prior to joining housing top 10 and housing bottom 12, all components of the nebulizer may be sterilized. Once the unit has been sterilized, medication 16 may be introduced into housing bottom 12. Finally, housing top 10 is connected to housing bottom 12 at housing seal 14, which is hermetically sealed by glue, sonic welding, or other known sealing techniques, to form nebulizer body 36.

To begin a medication therapy session with the pre-filled, small-volume nebulizer the patient or clinician may observe outlet port cap medication label 34 in order to verify the proper medicine is being used. Outlet port cap 26 may be removed, discarded, and replaced with either a mouthpiece attachment or some other type of patient interface common to the industry. To facilitate use of standard patient interface devices, outlet port 24 is of a shape, dimension and/or configuration commonly used in the industry. More specifically, outlet port 24 is preferably a generally cylindrical, tube having an outside diameter of between 15 and 30 millimeters, preferably between 20 and 25 millimeters, and most preferably of approximately 22 millimeters. Inlet port cap 30 may be removed and a source of gas under pressure such as an oxygen tube is connected to the inlet. During these maneuvers, pre-filled unit-dose of medication 16 within the nebulizer is prevented from exiting through inlet port 28 by one-way valve 32. As the therapy begins, gas under pressure enters inlet port 28 and travels through siphon 18 creating an area of relatively low pressure, which entrains at least a portion of pre-filled unit-dose of medication 16. The gas/medication mixture exits through jet 20, which directs the mixture such that it impinges against baffle 22 where the liquid medicine is broken up into small aerosol particles. The aerosol exits outlet port 24 and is delivered to the patient through a patient interface (not shown).

FIG. 2 depicts a side view of an alternate embodiment of the pre-filled, small-volume nebulizer of the present invention. In this embodiment outlet port 24 is sealed by piercable outlet port cap 38, which may either be removed or may be pierced at the time of use. In a preferred embodiment, the patient interface may be equipped with a mechanical appendage such as a spike which may be used to pierce outlet port cap 38 such that pre-filled unit-dose of medication 16 may be accessed without an operator or patient touching outlet port 24, thereby further reducing the likelihood of contamination. Furthermore, patient interface (not shown) may comprise a mouthpiece connected to a mouthpiece "T" which contains a spike for the purpose of saving time in the procedure of preparing for a therapy session. Outlet port 24 also contains an outlet port one-way valve 40, which allows aerosolized medication to flow out, but prevents retrograde flow in order to help defend against contamination.

The embodiment depicted in Fig. 2 further displays an alternate embodiment of the medication contained in the nebulizer. Specifically, in the embodiment depicted in Fig. 2, housing bottom 12 contains a plurality of pre-filled unit-doses of medication 16, and unit-dose completion demarcation marks 42. By providing a nebulizer body 36 pre-filled with multiple unit-doses of medicine, this embodiment of the present invention allows a patient or clinician to utilize the device for a predetermined period of time, twenty-four hours for example, without cleaning and reusing, and without disposing of the device earlier than is needed to prevent contamination. Unit-dose completion demarcation marks 42, allow a patient or clinician to determine when the delivery of a unit-dose of medication is complete and stop the therapy until it is time for the next.

FIG. 3 is a side view of a further alternate embodiment of the pre-filled, small-volume nebulizer of the present invention. In this embodiment, a compartment is provided within outlet port cap 26 for keeping a first component of a multi-component medication separate from a second component of a multi-component medication until use. This embodiment may find greatest application when the medicine to be administered is a mixture of one or more components, for example the mixture of Albuterol Sulfate and Ipratropium Bromide. However, mixing of medications can lead to additional problems associated with improper dosing and contamination. In some instances, it is believed that the useable life of these medicines once mixed is undesirably short. Therefore, in practice a patient or clinician generally mixes the medicines immediately prior to treatment. Of course, the increased handling of the medicine by a patient or clinician required during mixing may increase the likelihood of contamination and/or improper dosing. Therefore, by providing a pre-filled nebulizer which can separate multiple medication components until treatment, this embodiment of the present invention may greatly reduce these risks.

As shown in Fig. 3, outlet port cap 26 includes medication separation compartment 44, which may house a first component of a multi-component medication to be mixed with at least a second component of a multi-component medication at the time of use. Medication separation compartment 44 may be fabricated from a soft, malleable plastic composition such as a formulation of low density polyethylene. At the bottom of medication separation compartment 44 is a medication separation outlet gate 46 which is formed by fabricating a weak or thin portion of the plastic. As pressure is exerted at the top of medication separation compartment 44, medication separation outlet gate 46 breaks open and deposits its contents into housing bottom 12 where it mixes with pre-filled unit-dose of medication 16.

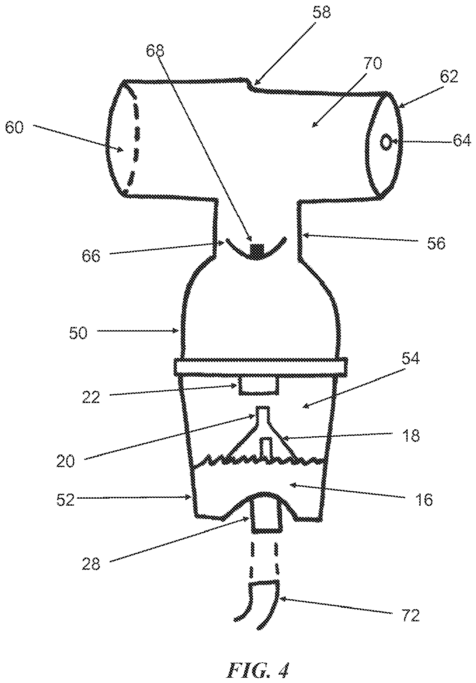

FIG. 4 shows a side view of an embodiment of the valve system for small-volume nebulizers. Each component depicted herein may be fabricated with any variety of plastic compounds, such as polypropylene, or any compound with appropriate characteristics for housing and delivering aerosolized medication. The components may be manufactured by means of injection molding or any other means of manufacture.

In this embodiment, the small-volume nebulizer 50 includes a medication reservoir 52 (sometimes referred to as a housing bottom), a aerosolizing chamber 54, a chimney 56 and a horizontal tube 58. Within the medication reservoir 52 and the aerosolizing chamber 54 of the small-volume nebulizer 50 are a baffle 22, a jet 20, and a siphon 18. The small-volume nebulizer 50 also includes a gas port 28. In the embodiment shown, medication 16 is also depicted in the medication reservoir 52. In some embodiments, the small-volume nebulizer 50 may be pre-filled with medication 16 as discussed further above. In such embodiments, the patient opening 60, the flow restrictor 64 and the gas port 28 may be hermetically sealed prior to use.

The horizontal tube 58 includes a patient opening 60 at one end and an ambient port 62 at a second end. Within the ambient port 62 is a flow restrictor 64 to choke the ambient airflow during the patient's breathing cycle. The patient opening 60 may be a typical size, such as 22 mm, to allow it to connect directly to a patient mouthpiece or to be adaptable to other patient connections, such as tracheostomies and ventilator circuits. Ambient port 62 may also be a typical size to allow it to be connectable to a filter or ventilator circuit.

In this embodiment, within the chimney 56 is at least one valve gate 66 held in place by a valve gate center pivot 68. The valve gate 66 separates the aerosolizing chamber 54 of the small-volume nebulizer 50 from a proximal cavity 70 of the small-volume nebulizer 50. In this embodiment, the proximal cavity 70 is the open space within the horizontal tube 58 between the patient opening 60 and the ambient port 62 extending into the chimney 56 to the top of the valve gate 66. In the embodiment shown, the valve gate 66 is located near the bottom of chimney 56. In some embodiments, the valve gate may be located proximate to the top of the chimney 56. One skilled in the art will recognize that the placement of the valve gate 66 within the chimney 56 will vary and remain within the scope of the disclosure.

During operation, high-pressure gas is introduced into the gas port 28 from gas source tube 72 which is connected prior to use. Gas flows into the gas port 28 at an appropriate flow rate, typically ranging from 6 to 10 liters per minute, and is directed through jet 20, which has a narrowed orifice in order to accelerate the velocity of the gas. One skilled in the art will recognize that the gas will typically be oxygen, air and/or another gas and remain within the scope of the disclosure. In some embodiments, the gas will be provided by a compressed gas source. As the velocity increases, pressure within siphon 18 drops creating a suction, which serves to entrain medication 16. Medication 16 is hurled as spray against baffle 22. Baffle 22 is a surface that causes large particles to fall out of suspension, thus reducing the overall average particle size of the aerosol. After the sprayed medication encounters the baffle 22, the remaining particles are suspended within the body of the aerosolizing chamber 54 until at least one of the gates 66, located within chimney 56 and stabilized by valve gate center pivot 68, opens in order to allow egress of the aerosol particles from aerosolizing chamber 54 and into proximal cavity 70.

The valve gate 66 and the flow restrictor 64 in combination operate as a valve system for the small-volume nebulizer 50 providing physiotherapy and medicated therapy to a user with a single device. The valve system is in communication with the aerosolizing chamber 54 and includes the valve gate 66 positioned between the aerosolizing chamber 54 and the patient opening 60.

Valve gates 66 are made of an appropriate substance, such as neoprene, which has the qualities of being lightweight, flexible, and impervious to liquid. Valve gates 66 are in a normally closed position until forced open by a drop in pressure within proximal cavity 70. This drop in pressure is the result of a combination of events. First, as the patient inhales gas is drawn through the proximal cavity 70 from the atmosphere through flow restrictor 64. Based upon the restricted airflow, a vacuum or negative pressure effect is created within the proximal cavity 70. Flow restrictor 64 is an apparatus designed to choke or limit the airflow through the ambient port 62. In some embodiments, the flow restrictor 64 is an orifice within a circular plate fixed into ambient port 62, which may also be referred to as an orifice plate. To accomplish different flows and pressures various sizes of orifices may be used or a single orifice with an adjustable size may be used as well.

Orifice plates utilize Bernoulli's principal, which states that there is a relationship between the pressure and velocity of a gas or fluid. As velocity increases, pressure decreases and vice versa. As ambient gas is pulled through the orifice plate by the inhalation effort of the patient, the ambient gas converges in order to travel through the small orifice, and in turn increases in velocity. The increased velocity reduces the surrounding pressure, which assists in opening valve gates 66. Additional embodiments may utilize in the place of flow restrictor 64 a venturi and/or an adjustable spring restrictor (such as a Threshold™ PEP device). Additionally, an additional gas source may be introduced into proximal cavity 70 that utilizes a Coanda effect to assist in changing the pressures in response to the patient breathing effort.

The Coanda effect is the tendency of a fluid to be attracted to a nearby surface. Thus, flow from an additional gas source could increase or decrease pressure and flow as needed in response to a patient's inhalation or exhalation effort. With an additional gas delivery spout that presented dual curves, one toward patient opening 60 and one toward ambient port 62, the direction of this gas would be influenced by at least one of the inhalation going toward patient opening 60 and the exhalation going toward ambient port 62.

In some embodiments, ambient port 62 may be a suitable size within the industry, for example 22 mm OD (outside diameter) or 22 mm ID (inside diameter), to accommodate a respiratory filter such as those of common knowledge in the art. In some embodiments, such a respiratory filter may be used in conjunction with a flow restrictor 64 to filter the airflow into and/or out of the proximal cavity 70. In some embodiments, a respiratory filter may operate as a flow restrictor 64 due to airflow characteristics associated with the respiratory filter.

In addition, aerosol particle size is effected by at least one of the source gas flow rate, viscosity of the medication, size of the jet orifice, shape of the reservoir, number and characteristics the baffle(s), and subjecting the aerosol to a substantially tortuous pathway. The valve gate 66 in the open position during inhalation serves to create a tortuous pathway for the aerosol to travel as it exits from aerosolizing chamber 54. As the valve gate 66 is substantially closed during exhalation, it serves to substantially restrict egress of aerosol from aerosolizing chamber 54 and acts as an additional baffle. Accordingly, the valve gate 66 assists in making the medicated aerosol particles more monodispersed.

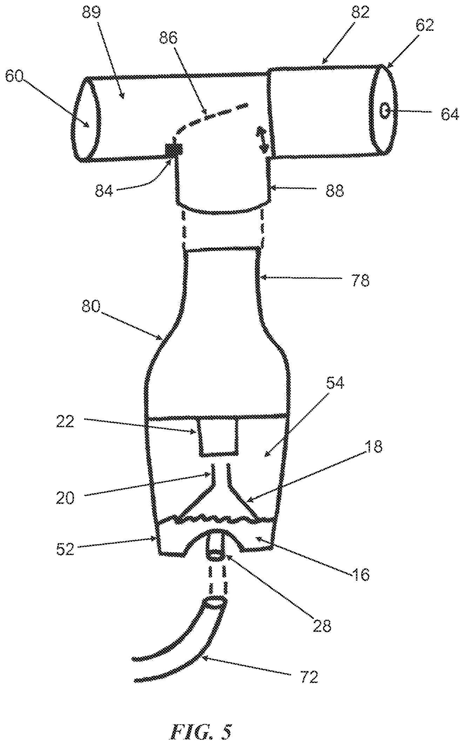

FIG. 5 is a side view of another embodiment of a small-volume nebulizer 80 and a "T" piece 82 that is connectable to the small-volume nebulizer 80. As discussed herein, each component depicted herein may be fabricated with any variety of plastic compounds, such as polypropylene, or any compound with appropriate characteristics for housing and delivering aerosolized medication. The components may be manufactured by means of injection molding or any other means of manufacture.

Similar to other embodiments described herein, the small-volume nebulizer 80 includes a medication reservoir 52, an aerosolizing chamber 54 and an aerosol output port 78 (also referred to as a chimney or outlet port). Within the medication reservoir 52 and the aerosolizing chamber 54 of the small-volume nebulizer 70 are a baffle 22, a jet 20, and a siphon 18. The small-volume nebulizer 80 also includes a gas port 28. In the embodiment shown, medication 16 is also depicted in the medication reservoir 52. In some embodiments, the small-volume nebulizer 80 may be pre-filled with medication 16 as discussed further above. In such embodiments, the aerosol output port 78 and the gas port 28 may be hermetically sealed prior to use.

In this embodiment, the "T" piece 82 includes a patient opening 60 at one end, an ambient port 62 at the end opposite from the patient opening 60 and a vertical "T" port 88 directed downward. Within the ambient port 62 is a flow restrictor 64 to choke the airflow during the patient's breathing cycle. In this embodiment, a valve gate 86 held in place by a valve gate side pivot 84 is located near the top of the vertical "T" port 88. In some embodiments, the valve gate 86 may be located proximate to the bottom of the vertical "T" port 88. One skilled in the art will recognize that the placement of the valve gate 86 within the vertical "T" port 88 will vary and remain within the scope of the disclosure.

The vertical "T" port 88 is designed to connect to the aerosol output port 78 of the small-volume nebulizer 80. In some embodiments, the vertical "T" port 88 is dimensioned to create a fitted connection with the top of aerosol output port 78. One skilled in the art will recognize that the vertical "T" port 88 may connect to the aerosol output port 78 in a variety of manners and remain within the scope of the disclosure. In some embodiments, the vertical "T" port 88 may include a spike or other mechanism to open a hermetic seal covering the top of the aerosol output port 78.

When the vertical "T" port 88 is connected to the aerosol output port 78, the valve gate 86 separates the aerosolizing chamber 54 of the small-volume nebulizer 80 from a proximal cavity 89 of the "T" piece 82. In this embodiment, the proximal cavity 89 is the area within the "T" piece 82 between the patient opening 60 and the ambient port 62 and above the top of the valve gate 86.

As discussed elsewhere herein, during operation, high-pressure gas is introduced into the gas port 28 from gas source tube 72 which is connected prior to use. Gas flows into the gas port 28 at an appropriate flow rate, typically ranging from 6 to 10 liters per minute, and is directed through jet 20, which has a narrowed orifice in order to accelerate the velocity of the gas. As the velocity increases, pressure within siphon 18 drops creating a suction, which serves to entrain medication 16. Medication 16 is hurled as spray against baffle 22. Baffle 22 is a surface that causes large particles to fall out of suspension, thus reducing the overall average particle size of the aerosol. After the sprayed medication encounters the baffle 22, the remaining particles are suspended within the body of the aerosolizing chamber 54 until the valve gate 86, located within vertical "T" port 88 and stabilized by valve gate side pivot 86, opens in order to allow egress of the aerosol particles from aerosolizing chamber 54 and into proximal cavity 89.

The valve gate 86 and the flow restrictor 64 in combination operate as a valve system for the small-volume nebulizer 80 providing physiotherapy and medicated therapy to a user with a single device. The valve system is in communication with the aerosolizing chamber 54 and includes the valve gate 86 positioned between the aerosolizing chamber 54 and the patient opening 60.

As discussed above, valve gate 86 is made of an appropriate substance, such as neoprene, which has the qualities of being lightweight, flexible, and impervious to liquid. The valve gate 86 is in a normally closed position until forced open by a drop in pressure within proximal cavity 89. This drop in pressure is the result of a combination of events. First, as the patient inhales gas is drawn through the proximal cavity 89 from the atmosphere through flow restrictor 64. Based upon the restricted airflow, a vacuum or negative pressure effect is created within the proximal cavity 89. Flow restrictor 64 is an apparatus designed to choke or limit the airflow through the ambient port 62. In some embodiments, the flow restrictor 64 is an orifice within a circular plate fixed into ambient port 62, which may also be referred to as an orifice plate. To accomplish different flows and pressures various sizes of orifices may be used or a single orifice with an adjustable size may be used as well.

As ambient gas is pulled through the orifice plate by the inhalation effort of the patient, the ambient gas converges in order to travel through the small orifice, and in turn increases in velocity. The increased velocity reduces the surrounding pressure, which assists in opening valve gate 86. In some embodiments, the flow restrictor 64 may be replaced by a venturi and/or an adjustable spring restrictor (such as a Threshold™ PEP or Threshold® IMT device) to regulate inspiratory and/or expiratory flow. Additionally, an additional gas source may be introduced into proximal cavity 89 that utilizes a Coanda effect to assist in changing the pressures in response to the patient breathing effort.

The flow from an additional gas source could increase or decrease pressure as needed in response to a patient's inhalation or exhalation effort. With an additional gas delivery spout that presented dual curves, one toward patient opening 60 and one toward ambient port 62, the direction of this gas would be influenced by at least one of the inhalation going toward patient opening 60 and the exhalation going toward ambient port 62.

In some embodiments, ambient port 62 may be a suitable size within the industry, for example 22 mm OD, to accommodate a respiratory filter such as those of common knowledge in the art. In some embodiments, such a respiratory filter may be used in conjunction with a flow restrictor 64 to filter the airflow into and/or out of the proximal cavity 89. In some embodiments, a respiratory filter may operate as a flow restrictor 64 due to airflow characteristics associated with the respiratory filter.

In addition, aerosol particle size is effected by at least one of the source gas flow rate, viscosity of the medication, size of the jet orifice, shape of the reservoir, number and characteristics the baffle(s), and subjecting the aerosol to a substantially tortuous pathway. The valve gate 86 in the open position during inhalation serves to create a tortuous pathway for the aerosol to travel as it exits from aerosolizing chamber 54. As the valve gate 86 is substantially closed during exhalation, it serves to substantially restrict egress of aerosol from aerosolizing chamber 54 and acts as an additional baffle. Accordingly, the valve gate 86 assists in making the medicated aerosol particles more monodispersed.

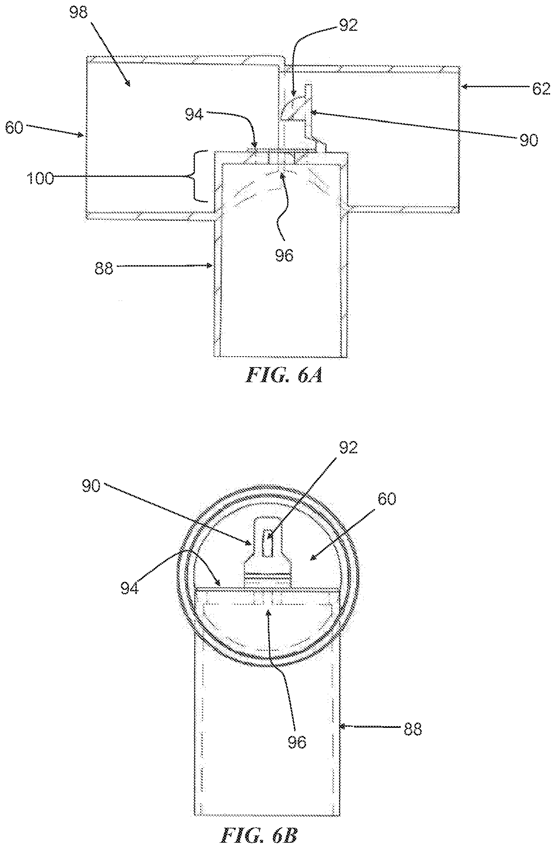

FIG. 6A and FIG. 6B different views of another embodiment of a "T" piece with the valve system with the valve gate center pivot 90 in an open position. FIG. 6A is a side view of the "T" piece and FIG. 6B is a frontal view of the "T" piece. In some embodiments, the design disclosed herein may be integrated into a small-volume nebulizer. For example, the vertical "T" port 88 may instead comprise the chimney of a small-volume nebulizer. As discussed herein, each component depicted herein may be fabricated with any variety of plastic compounds, such as polypropylene, or any compound with appropriate characteristics for housing and delivering aerosolized medication. The components may be manufactured by means of injection molding or any other means of manufacture.

In this embodiment, the "T" piece includes a patient opening 60 at one end, an ambient port 62 at the end opposite from the patient opening 60 and a vertical "T" port 88 directed downward. In some embodiments, a flow restrictor to choke the airflow during the patient's breathing cycle may be located within the ambient port 62. In this embodiment, a valve gate 94 may be held in place by a valve gate center pivot 90 which is shown in an open position. As discussed above, valve gate 94 is made of an appropriate substance, such as neoprene, which has the qualities of being lightweight, flexible, and impervious to liquid.

The valve gate 94 is located near the top of the vertical "T" port 88. In some embodiments, the valve gate 94 may be located proximate to the bottom of the vertical "T" port 88. One skilled in the art will recognize that the placement of the valve gate 94 within the vertical "T" port 88 will vary and remain within the scope of the disclosure.

The vertical "T" port 88 is designed to connect to an aerosol output port of a small-volume nebulizer. One skilled in the art will recognize that the vertical "T" port 88 may connect to the aerosol output port in a variety of manners and remain within the scope of the disclosure. As discussed elsewhere herein, the vertical "T" port 88 may include a spike or other mechanism to open a hermetic seal covering the top of an aerosol output port. When the vertical "T" port 88 is connected to an aerosol output port, the valve gate 94 separates the aerosolizing chamber of a small-volume nebulizer from a proximal cavity 98 of the "T" piece. In this embodiment, the proximal cavity 98 is the area within the "T" piece between the patient opening 60 and the ambient port 62 and above the top of the valve gate 94.

In the embodiment shown, the valve gate center pivot 90 is shown in the open position. Prior to administering a therapy using the "T" piece, the valve gate center pivot 90 will be latched into a closed position. In some embodiments, the valve gate center pivot 90 will be latched during manufacture of the "T" piece. In this embodiment, the valve gate center pivot 90 includes a tab 92. When closed, the tab 92 passes through the valve gate 94 and engages with a slot 96 built in to a mount located in this embodiment at the top of the vertical "T" port 88. The structure for the mount design includes the slot 96 for engagement with the tab 92 of the valve gate center pivot 90 and one or more openings under the valve gate 94. As discussed further herein, medicated aerosol from an attached small-volume nebulizer passes through the openings when the valve gate 94 opens in conjunction with a patient's inhalation. When the valve gate 94 closes in conjunction with a patient's exhalation, the valve gate 94 creates a seal with the mount. In some embodiments, the mount and valve gate 94 may be located at other locations within the vertical "T" port 88.

The connection created through engagement of the tab 92 with the slot 96 is designed to hold the valve gate 94 in place during operation of the "T" piece. Accordingly, the connection must be sufficient to withstand the various effects of the patient's breathing cycle, the pressures created within the attached small-volume nebulizer and any other effects created during the set-up and operation of the device. One skilled in the art will recognize that various connections may be employed which meet the operational necessities of the connection, such as a friction connection, a snap-connection, a locking connection, adhesives, fitted connections, pin connections, and other connections.

In this embodiment, the "T" piece is depicted as a "drool T" and is designed with a raised section 100 which limits the ability of a patient's drool to feed back into the small-volume nebulizer through the valve gate 94 and the vertical "T" port 88. One skilled in the art will recognize that other designs may be operable to provide anti-drool or drool catching characteristics and remain within the scope of the disclosure.

As discussed elsewhere herein, during operation, medication is aerosolized within a small-volume nebulizer and the particles remain suspended within the body of the small-volume nebulizer until the valve gate 94 opens in order to allow egress of the aerosol particles from the small-volume nebulizer and into proximal cavity 98. The valve gate 94 limits the escape of medication during exhalation conserving medicine. The valve gate 94 is in a normally closed position until forced open by a drop in pressure within proximal cavity 98. In some embodiments, this drop in pressure is the result of a combination of events. First, as the patient inhales gas is drawn through the proximal cavity 98 from the atmosphere through a flow restrictor. Based upon the restricted airflow, a vacuum or negative pressure effect is created within the proximal cavity 98. A flow restrictor is an apparatus designed to choke or limit the airflow through the ambient port 62. To accomplish different flows and pressures various designs of flow restrictors may be utilized. In some embodiments, a series of interchangeable flow restrictors which are compatible with the ambient port 62 may be available to customize the flow and pressure characteristics.

As ambient gas is pulled through the flow restrictor by the inhalation effort of the patient, the ambient gas converges in order to travel through the flow restrictor, and in turn increases in velocity. The increased velocity reduces the surrounding pressure, which assists in opening valve gate 94. In some embodiments, the flow restrictor may be replaced by a venturi and/or an adjustable spring restrictor (such as a Threshold™ PEP or Threshold® IMT device) regulate inspiratory and/or expiratory flow.

In some embodiments, an additional gas source may be introduced into proximal cavity 98 that utilizes a Coanda effect to assist in changing the pressures in response to the patient breathing effort. The flow from an additional gas source could increase or decrease pressure as needed in response to a patient's inhalation or exhalation effort. With an additional gas delivery spout that presented dual curves, one toward patient opening 60 and one toward ambient port 62, the direction of this gas would be influenced by at least one of the inhalation going toward patient opening 60 and the exhalation going toward ambient port 62.

In some embodiments, ambient port 62 may accommodate a respiratory filter such as those of common knowledge in the art. Such a respiratory filter may be used in conjunction with a flow restrictor to filter the airflow into and/or out of the proximal cavity 98. In some embodiments, a respiratory filter may operate as a flow restrictor due to airflow characteristics associated with the respiratory filter.

In addition, aerosol particle size is effected by at least one of the source gas flow rate, viscosity of the medication, size of the jet orifice, shape of the reservoir, number and characteristics the baffle(s), and subjecting the aerosol to a substantially tortuous pathway. The valve gate 98 in the open position during inhalation serves to create a tortuous pathway for the aerosol to travel as it exits from a connected small-volume nebulizer. As the valve gate 94 is substantially closed during exhalation, it serves to substantially restrict egress of aerosol from a connected small-volume nebulizer and acts as an additional baffle. Accordingly, the valve gate 94 assists in making the medicated aerosol particles more monodispersed.

The invention being thus described and further described in the claims, it will be obvious that the same may be varied in many ways. Such variations are not to be regarded as a departure from the scope of the invention and all such modifications as would be obvious to one skilled in the art are intended to be included within the scope of the apparatus, system, process and computer program product described.

## Claims

1. A valve system apparatus (64, 66; 64, 86; 64, 94) for attachment to a small-volume nebulizer (50; 80),
said valve system apparatus (64, 66; 64, 86; 64, 94) comprising:
a valve system body comprising:
a vertical port (56; 88) configured to connect with an aerosol output port (78) of said small-volume nebulizer (50; 80),
wherein contained within said vertical port (56; 88) is a valve gate (66; 86; 94) which is configured to open based upon an inhalation by a user and which is configured to close based upon an exhalation by the user; and
a patient interface tube (58; 82) at the top of said vertical port (56; 88),
wherein the patient interface tube (58; 82) includes a first end having a patient opening (60) operable to connect with a patient interface component and a second end having a flow restrictor (64) comprises an orifice plate and within an ambient port (62),
wherein said flow restrictor (64) is configured to choke or limit the airflow through the ambient port (62) thereby choking the flow of the user's inspiratory and expiratory effort

2. The apparatus (64, 66; 64, 86; 64, 94) according to Claim 1, wherein said patient interface component comprises a mouthpiece.

3. The apparatus (64, 66; 64, 86; 64, 94) according to claim 2, wherein said orifice plate is in a removable connection with said ambient port (62) and is one of a plurality of orifice plates, each providing distinct airflow characteristics, or wherein said orifice plate comprises an orifice with an adjustable size.

4. The apparatus (64, 66; 64, 86; 64, 94) according to any one of the preceding claims, wherein said flow restrictor (64) comprises a respiratory filter.

5. The apparatus (64, 66; 64, 86; 64, 94) according to any one of the preceding claims, further comprising a respiratory filter in a removable connection with said flow restrictor (64).

6. The apparatus (64, 66; 64, 86; 64, 94) according to any one of the preceding claims, wherein said valve gate (66; 86; 94) comprises a flexible material attached to a mount located within said vertical port (88) by a valve gate center pivot (90) which is engaged with said mount.

7. A small-volume nebulizer (50; 80) comprising the valve system apparatus (64, 66; 64, 86; 64, 94) of any one of the preceding claims,
the small-volume nebulizer (50; 80) having:
a small-volume nebulizer body comprising:
an aerosolizing chamber (54),
wherein contained within said aerosolizing chamber (54) is at least one of a siphon (18), a jet (20), and a baffle (22),
an aerosol output port (78) located at the top of said aerosolizing chamber (54) and connected to said vertical port (56; 88), and
an inlet opening (28) operable to connect with a gas input (72);
wherein, during operation of the small-volume nebulizer (50; 80) by a user, medication (16) is aerosolized within said aerosolizing chamber (54) and remains within said aerosolizing chamber (54) until said valve gate (66; 86; 94) opens based upon said inhalation by the user, and said valve gate (66; 86; 94) closes with said exhalation by the user, suppressing the egress of aerosolized medication (16) from said aerosolizing chamber (54).

8. The small-volume nebulizer (50; 80) according to claim 7, further comprising:
a first seal removably engaged with said patient opening (60);
a second seal removably engaged with said inlet opening (28);
a third seal removably engaged with said ambient port (62); and
a unit-dose of medication (16) contained within said small-volume nebulizer body.

9. The small-volume nebulizer (50; 80) according to claim 8, further comprising a plurality of said unit-doses of medication (16) and optionally unit-dose completion demarcation marks (42).

10. The small-volume nebulizer (50; 80) according to claim 8, wherein at least one of said first seal, said second seal and said third seal is comprised of a piercable seal, and/or wherein preferably said flow restrictor (64) is fixed within said ambient port (62) and said third seal covers the flow restrictor.

11. The apparatus (64, 66; 64, 86; 64, 94) according to any one of claims 1 to 6, further comprising a seal opening apparatus, wherein connecting said valve system apparatus (64, 66; 64, 86; 64, 94) to said small-volume nebulizer (50; 80) operates to open a seal associated with the aerosol output port (56; 78) of the small-volume nebulizer.

## Patentansprüche

1. Eine Ventilsystemvorrichtung (64, 66; 64, 86; 64, 94) zur Anbringung an einen Geringvolumen-Zerstäuber (50; 80),
wobei die Ventilsystemvorrichtung (64, 66; 64, 86; 64, 94) Folgendes aufweist:
einen Ventilsystemkörper, der Folgendes aufweist:
einen vertikalen Anschluss (56; 88), der zu einer Verbindung mit einem Aerosol-Ausgabeanschluss (78) des Geringvolumen-Zerstäubers (50; 80) eingerichtet ist,
wobei im vertikalen Anschluss (56; 88) ein Sperrventil (66; 86; 94) enthalten ist, das eingerichtet ist, sich basierend auf einer Einatmung durch einen Nutzer zu öffnen, und das eingerichtet ist, sich basierend auf einer Ausatmung durch den Nutzer zu schließen; und
einen Patientenschnittstellentubus (58; 82) an der Oberseite des vertikalen Anschlusses (56; 88),
wobei der Patientenschnittstellentubus (58; 82) ein erstes Ende mit einer Patientenöffnung (60), die zum Verbinden mit einer Patientenschnittstellenkomponente betreibbar ist, und ein zweites Ende mit einem Durchflussbegrenzer (64) innerhalb eines Umgebungsanschlusses (62) aufweist,
wobei der Durchflussbegrenzer (64) eine Blendenplatte aufweist und eingerichtet ist, den Luftstrom durch den Umgebungsanschluss (62) zu drosseln oder zu begrenzen, wodurch der Strom der Ein- und Ausatmungsbemühung des Nutzers gedrosselt wird.

2. Die Vorrichtung (64, 66; 64, 86; 64, 94) nach Anspruch 1, wobei die Patientenschnittstellenkomponente ein Mundstück aufweist.

3. Die Vorrichtung (64, 66; 64, 86; 64, 94) nach Anspruch 2, wobei die Blendenplatte in einer entfernbaren Verbindung mit dem Umgebungsanschluss (62) steht und eine von einer Vielzahl von Blendenplatten ist, die jeweils unterschiedliche Luftströmungseigenschaften zur Verfügung stellt, oder wobei die Blendenplatte eine Öffnung mit einer einstellbaren Größe aufweist.

4. Die Vorrichtung (64, 66; 64, 86; 64, 94) nach einem der vorhergehenden Ansprüche, wobei der Durchflussbegrenzer (64) einen Atemfilter aufweist.

5. Die Vorrichtung (64, 66; 64, 86; 64, 94) nach einem der vorhergehenden Ansprüche, die ferner einen Atemfilter in einer abnehmbaren Verbindung mit dem Durchflussbegrenzer (64) aufweist.

6. Die Vorrichtung (64, 66; 64, 86; 64, 94) nach einem der vorhergehenden Ansprüche, wobei das Sperrventil (66; 86; 94) ein flexibles Material aufweist, das an eine Halterung angebracht ist, die innerhalb des vertikalen Anschlusses (88) mittels eines Sperrventilzentrierungszapfens (90) angeordnet ist, der mit der Halterung in Eingriff steht.

7. Geringvolumen-Zerstäuber (50; 80), der die Ventilsystemvorrichtung (64, 66; 64, 86; 64, 94) nach einem der vorhergehenden Ansprüche aufweist, wobei der Geringvolumen-Zerstäuber (50; 80) Folgendes aufweist:
einen Geringvolumen-Zerstäuberkörper, der Folgendes aufweist:
eine Aerosolisierungskammer (54),
wobei in der Aerosolisierungskammer (54) ein Siphon (18) und/oder eine Düse (20) und/oder ein Stromstörer (22) enthalten ist,
einen Aerosol-Ausgabeanschluss (78), der über der Aerosolisierungskammer (54) angeordnet und mit dem vertikalen Anschluss (56; 88) verbunden ist, und
eine Einlassöffnung (28), die zum Verbinden mit einem Gaseingang (72) betreibbar ist;
wobei, während des Betriebs des Geringvolumen-Zerstäubers (50; 80) durch einen Nutzer, ein Medikament (16) innerhalb der Aerosolisierungskammer (54) aerosolisiert wird und innerhalb der Aerosolisierungskammer (54) verbleibt, bis sich das Sperrventil (66; 86; 94) öffnet, und zwar basierend auf der Einatmung durch den Nutzer, und wobei sich das Sperrventil (66; 86; 94) mit der Ausatmung durch den Nutzer schließt und dabei den Austritt von aerosolisiertem Medikament (16) aus der Aerosolisierungskammer (54) unterdrückt.

8. Der Geringvolumen-Zerstäuber (50; 80) nach Anspruch 8, der ferner Folgendes aufweist:
eine erste Dichtung, die lösbar mit der Patientenöffnung (60) in Eingriff steht;
eine zweite Dichtung, die lösbar mit der Einlassöffnung (28) in Eingriff steht;
eine dritte Dichtung, die lösbar mit dem Umgebungsanschluss (62) in Eingriff steht; und
eine Einheitsdosis eines Medikaments (16), das in dem Geringvolumen-Zerstäuberkörper enthalten ist.

9. Geringvolumen-Zerstäuber (50; 80) nach Anspruch 8, der ferner eine Vielzahl der Einheitsdosen des Medikaments (16) und optional Vollständigkeitsmarkierungen (42) für Einheitsdosen aufweist.

10. Geringvolumen-Zerstäuber (50; 80) nach Anspruch 8, wobei die erste Dichtung und/oder die zweiten Dichtung und/oder die dritte Dichtung aus einer durchstechbaren Dichtung besteht, und/oder wobei vorzugsweise der Durchflussbegrenzer (64) innerhalb des Umgebungsanschlusses (62) befestigt ist und die dritte Dichtung den Durchflussbegrenzer abdeckt.

11. Die Vorrichtung (64, 66; 64, 86; 64, 94) nach einem der Ansprüche 1 bis 6, die ferner eine Dichtungsöffnungsvorrichtung aufweist, wobei Verbinden der Ventilsystemvorrichtung (64, 66; 64, 86; 64, 94) mit dem Geringvolumen-Zerstäuber (50; 80) zum Öffnen einer Dichtung wirkt, die dem Aerosol-Ausgabeanschluss (56; 78) des Geringvolumen-Zerstäubers assoziiert ist.

## Revendications

1. Appareil à système de valve (64, 66 ; 64, 86 ; 64, 94) destiné à être fixé à un nébuliseur à faible volume (50 ; 80), ledit appareil à système de valve (64, 66 ; 64, 86 ; 64, 94) comprenant :
un corps de système de valve comprenant :
une ouverture verticale (56 ; 88) configurée pour se connecter à une ouverture de sortie d'un aérosol (78) dudit nébuliseur à faible volume (50 ; 80),
dans lequel à l'intérieur de ladite ouverture verticale (56 ; 88) se trouve un obturateur de vanne (66 ; 86 ; 94) qui est configuré pour s'ouvrir sur la base d'une inhalation en cours par un utilisateur et qui est configuré pour se refermer sur la base d'une exhalation en cours par l'utilisateur ; et
un tube d'interface de patient (58 ; 82) en haut de ladite ouverture verticale (56 ; 88),
dans lequel le tube d'interface de patient (58 ; 82) comporte une première extrémité ayant une ouverture côté patient (60) qui peut être utilisée pour se connecter à un composant d'interface de patient et une deuxième extrémité ayant un réducteur de flux (64) dans une ouverture ambiante (62),
dans lequel ledit réducteur de flux (64) comprend une plaque à ouverture et est configuré pour étrangler ou limiter le flux d'air passant à travers l'ouverture ambiante (62) étranglant ainsi le flux de l'effort d'inspiration ou d'expiration de l'utilisateur.

2. Appareil (64, 66 ; 64, 86 ; 64, 94) selon la revendication 1, dans lequel ledit composant d'interface de patient comprend un embout buccal.

3. Appareil (64, 66 ; 64, 86 ; 64, 94) selon la revendication 2, dans lequel ladite plaque à ouverture est connectée de façon amovible à ladite ouverture ambiante (62) et est une parmi une pluralité de plaques à ouverture, chacune fournissant des caractéristiques différentes de flux d'air, ou dans lequel ladite plaque à ouverture comprend une ouverture ayant une taille réglable.

4. Appareil (64, 66 ; 64, 86 ; 64, 94) selon l'une quelconque des revendications précédentes, dans lequel ledit réducteur de flux (64) comprend un filtre respiratoire.

5. Appareil (64, 66 ; 64, 86 ; 64, 94) selon l'une quelconque des revendications précédentes, comprenant en outre un filtre respiratoire connecté de façon amovible avec ledit réducteur de flux (64).

6. Appareil (64, 66 ; 64, 86 ; 64, 94) selon l'une quelconque des revendications précédentes, dans lequel ledit obturateur de vanne (66 ; 86 ; 94) comprend un matériau flexible fixé à un support placé dans ladite ouverture verticale (88) par un pivot central d'obturateur de vanne (90) qui est engagé dans ledit support.

7. Nébuliseur à faible volume (50 ; 80) comprenant un appareil à système de valve (64, 66 ; 64, 86 ; 64, 94) selon l'une quelconque des revendications précédentes,
le nébuliseur à faible volume (50 ; 80) ayant :
un corps de nébuliseur à faible volume comprenant :
une chambre d'aérosolisation (54),
dans lequel à l'intérieur de ladite chambre d'aérosolisation (54) se trouve au moins un parmi un siphon (18), un jet (20), et un déflecteur (22),
une ouverture de sortie d'aérosol (78) placée au sommet de ladite chambre d'aérosolisation (54) et connectée à ladite ouverture verticale (56 ; 88), et
une ouverture d'admission (28) qui peut être utilisée pour se connecter à une entrée de gaz (72) ;
dans lequel, pendant le fonctionnement du nébuliseur à faible volume (50 ; 80) par un utilisateur, un médicament (16) est mis en aérosol dans ladite chambre d'aérosolisation (54) et reste dans ladite chambre d'aérosolisation (54) jusqu'à ce que ladite plaque à ouverture (66 ; 86 ; 94) s'ouvre sur la base de ladite inhalation par l'utilisateur, et que ladite plaque à ouverture (66 ; 86 ; 94) se ferme sur la base de ladite expiration par l'utilisateur, supprimant la sortie du médicament mis en aérosol (16) de ladite chambre d'aérosolisation (54).

8. Nébuliseur à faible volume (50 ; 80) selon la revendication 7, comprenant en outre :
un premier joint engagé de façon amovible avec ladite ouverture côté patient (60) ;
un deuxième joint engagé de façon amovible avec ladite ouverture d'admission (28) ;
un troisième joint engagé de façon amovible avec ladite ouverture ambiante (62) ; et
une dose unitaire de médicament (16) contenue dans ledit corps de nébuliseur à faile volume.

9. Nébuliseur à faible volume (50 ; 80) selon la revendication 8, comprenant en outre une pluralité desdites doses unitaires de médicament (16) et en option des marques de démarcation de fin de dose unitaire (42).

10. Nébuliseur à faible volume (50 ; 80) selon la revendication 8, dans lequel au moins un parmi ledit premier joint, ledit deuxième joint et ledit troisième joint est constitué d'un joint pouvant être percé, et/ou dans lequel, de préférence, ledit réducteur de flux (64) est fixé dans ladite ouverture ambiante (62) et ledit troisième joint recouvre le réducteur de flux.

11. Appareil (64, 66 ; 64, 86 ; 64, 94) selon l'une quelconque des revendications 1 à 6, comprenant en outre un appareil d'ouverture de joint, dans lequel la connexion dudit appareil à système de valve (64, 66 ; 64, 86 ; 64, 94) audit nébuliseur à faible volume (50 ; 80) fonctionne pour ouvrir un joint associé à l'ouverture de sortie d'aérosol (56 ; 78) du nébuliseur à faible volume.
